# EUROPEAN PATENT SPECIFICATION

(11) **EP 1 965 757 B1**
(45) Date of publication and mention of the grant of the patent: **23.11.2016**
(21) Application number: 06842348.2
(22) Date of filing: 09.10.2006
(51) Int. Cl.: A61K 8/42, A61Q 5/00

(54) **COSMETIC USE OF A HYDROXYALKYLUREA AS AN AGENT FOR TREATING DESQUAMATIVE CONDITIONS OF THE SCALP; COSMETIC TREATMENT COMPOSITIONS AND PROCESSES**
KOSMETISCHE VERWENDUNG EINES HYDROXYALKYL-HARNSTOFFS ALS MITTEL ZUR BEHANDLUNG DESQUAMATIVER LEIDEN DER KOPFHAUT SOWIE ZUSAMMENSETZUNG UND VERFAHREN ZUR KOSMETISCHEN ANWENDUNG
UTILISATION COSMÉTIQUE D'UNE HYDROXYALKYLURÉE EN TANT QU'AGENT DE TRAITEMENT DE CONDITIONS DESQUAMANTES DU CUIR CHEVELU; COMPOSITIONS ET PROCÉDÉ DE TRAITEMENT COSMÉTIQUE

(30) Priority: 14.11.2005 FR 0553442; 22.11.2005 US 738563 P
(43) Date of publication of application: 10.09.2008
(73) Proprietor: L'Oréal, 75008 Paris (FR)
(72) Inventor: LEREBOUR, Géraldine, F-78350 Les Loges en Josas (FR)
(74) Representative: Dodin, Catherine
(86) International application number: PCT/IB2006/003912
(87) International publication number: WO 2007/054833

(56) References cited:
- EP-A- 0 500 352
- EP-A- 1 743 623
- WO-A-87/04617
- WO-A-03/086291

## Description

The present invention relates to the non-therapeutic use of a cosmetic composition comprising at least one hydroxyalkylurea of particular formula (1) as an agent for treating dandruff

The present invention also relates to the use as medicament of at least one hydroxyalkylurea of particular formula (1) for treating desquamative conditions of the scalp, in particular dandruff and seborrhoeic dermatitis.

The invention also relates to a cosmetic process for treating dandruff which consists in applying to the hair and/or the scalp a cosmetic composition comprising, in a physiologically acceptable medium, at least one hydroxyalkylurea of particular formula.

Desquamative disorders of the hair and/or the scalp such as dandruff or seborrhoeic dermatitis are associated in particular with the permanent presence of a yeast characteristic of the genus *Malassezia* (*ovalis, orbiculare* or *furfur*), this genus previously being known as *Pityrosporum* (*ovale* or *orbiculare*).

To combat desquamative disorders of the scalp and in particular dandruff or seborrhoeic dermatitis, it is known practice to use antifungal agents applied topically in various forms. These agents are directed, by virtue of their antifungal power, towards eliminating or controlling the multiplication of a resident yeast of the scalp, belonging to the genus *Malassezia* and its variants (*M. ovalis, M. orbiculare or M. furfur).*

Many agents are claimed, known and used for this purpose, among which mention may be made of zinc pyrithione, omadine, tars, triclosan, piroctone olamine, selenium disulfide and, more recently, tropolone and hinokitiol (Research Disclosure No. 429, January 2000).

The antifungal activity of these substances towards a yeast characteristic of the genus *Malassezia* is not sufficiently satisfactory. Some of these active agents have formulation problems on account of incompatibility with certain ingredients, for instance piroctone olamine. Most of them, after application to the scalp, do not provide any moisturizing power.

WO03/086291 describes a method of treating cosmetic and dermatological disorders with a composition comprising a molecular complex formed between urea and a functional substance comprising at least one hydroxyl group and one carbonyl group.

WO87/04617 describes a composition active for the treatment of hyperkeratotic skin diseases, seborrheic eczema, dermatomycosis and onychomycosis, thickened and chapped skin, comprising 40-80% of propylene glycol and/or polyethylene glycol, and 5-20% of urea.

There is thus still a need for novel active agents that simultaneously have good antifungal activity, allowing desquamative conditions of the scalp to be treated efficiently, and moisturizing power.

The Applicant has now discovered, surprisingly and unexpectedly, that hydroxyalkylurea compounds of formula (1) that will be defined in greater detail hereinbelow have good antifungal activity towards a yeast characteristic of the genus *Malassezia* and also moisturizing properties.

A first subject of the invention concerns the non-therapeutic use of a cosmetic composition comprising, in a physiologically acceptable medium, at least one hydroxyalkylurea corresponding to the general formula (1): 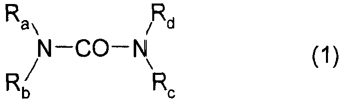 in which Rₐ, R_{b}, R_{c} and R_{d} each independently represent a hydrogen atom, a C₁-C₄ alkyl group or a C₂-C₆ hydroxyalkyl group possibly containing from 1 to 5 hydroxyl groups, at least one of the radicals Rₐ-R_{d} representing a hydroxyalkyl group, and also the salts, solvates and isomers thereof, as an agent for treating dandruff.

Another subject of the invention concerns a composition containing, in a physiologically acceptable medium, at least one hydroxyalkylurea of formula (1) or a salt and/or solvate and/or isomer thereof and at least one other active agent for combating desquamative conditions of the scalp and more particularly dandruff and seborrhoeic dermatitis. Another subject of the invention concerns the hydroxyalkylurea of formula (1) or a salt and/or solvate and/or isomer thereof, for its use as medicament for treating desquamative conditions of the scalp.

The invention also relates to a cosmetic treatment process for combating dandruff, which consists in applying to the hair and/or the scalp at least one composition comprising, in a physiologically acceptable medium, at least one hydroxyalkylurea of formula (1) or a salt and/or solvate and/or isomer thereof.

The term "physiologically acceptable medium" means a non-toxic medium that can be applied to the human scalp and hair.

In formula (1), among the alkyl groups, mention may be made especially of methyl, ethyl, n-propyl, isopropyl, n-butyl, isobutyl and tert-butyl groups.

The compounds of formula (1) that are preferred are those containing only one hydroxyalkyl group, i.e. those for which Rₐ is a hydroxyalkyl group and R_{b}, R_{c} and R_{d} represent, independently of each other, a hydrogen atom or a C₁-C₄ alkyl group. The compounds of formula (1) for which Rₐ is a hydroxyalkyl group and R_{b}, R_{c} and R_{d} each represent a hydrogen atom are more particularly preferred.

Among the hydroxyalkyl groups, those containing only one hydroxyl group and in particular hydroxyethyl, hydroxypropyl, hydroxybutyl, hydroxypentyl and hydroxyhexyl are preferred. The hydroxyethyl group is preferred.

Preferred compounds of formula (1) that may be mentioned include N-(2-hydroxyethyl)urea; N-(2-hydroxypropyl)urea; N-(3-hydroxypropyl)urea; N-(2,3-dihydroxypropyl)urea; N-(2,3,4,5,6-pentahydroxyhexyl)urea; N-methyl-N-(1,3,4,5,6-pentahydroxy-2-hexyl)urea; N-methyl-N'-(1-hydroxy-2-methyl-2-propyl)urea; N-(1-hydroxy-2-methyl-2-propyl)urea; N-(1,3-dihydroxy-2-propyl)urea; N-[tris(hydroxymethyl)-methyl]urea; N-ethyl-N'-(2-hydroxyethyl)urea; N,N-bis(2-hydroxyethyl)urea; N,N'-bis(2-hydroxyethyl)-urea; N,N-bis(2-hydroxypropyl)urea; N,N'-bis(2-hydroxypropyl)urea; N,N-bis(2-hydroxyethyl)-N'-propylurea; N,N-bis(2-hydroxypropyl)-N'-(2-hydroxyethyl)urea; N-tert-butyl-N'-(2-hydroxyethyl)-N'-(2-hydroxypropyl)-urea; N-(1,3-dihydroxy-2-propyl)-N'-(2-hydroxyethyl)-urea; N,N-bis(2-hydroxyethyl)-N',N'-dimethylurea; N,N,N',N'-tetrakis(2-hydroxyethyl)urea; and N',N'-bis(2-hydroxyethyl)-N',N'-bis(2-hydroxypropyl)urea.

A compound that is particularly preferred for use in the present invention is N-(2-hydroxyethyl)urea, which is referred to hereinbelow as "hydroxyethylurea".

The hydroxyalkylureas of formula (1) may be prepared as described especially in patent application DE-A-2 703 185. Among these, the hydroxyethylurea is also commercially available, in the form of a mixture at 50% by weight in water, from the company National Starch under the trade name Hydrovance®.

Salts of such compounds that may be mentioned include the salts of mineral acids, such as sulfuric acid, hydrochloric acid, hydrobromic acid, hydriodic acid, phosphoric acid and boric acid. Mention may also be made of the salts of organic acids, which may comprise one or more carboxylic acid, sulfonic acid or phosphonic acid groups. They may be linear, branched or cyclic aliphatic acids or alternatively aromatic acids. These acids may also comprise one or more hetero atoms chosen from O and N, for example in the form of hydroxyl groups. Mention may especially be made of propionic acid, acetic acid, terephthalic acid, citric acid and tartaric acid.

The term "solvate" means a stoichiometric mixture of the said compound of formula (1) with one or more water molecules or organic solvent molecules, such a mixture being derived from the synthesis of the compound of formula (1).

The hydroxyalkylureas in accordance with the invention are preferably present in the compositions in accordance with the invention in contents of from 0.01% to 50% by weight, more preferentially from 0.1% to 20% by weight and even more preferentially from 0.1% to 10% by weight relative to the total weight of the composition.

According to the invention, the desquamative conditions of the hair and/or the scalp are, for example, dandruff and seborrhoeic dermatitis. Preferably, the said desquamative conditions of the scalp are those induced by the yeast of the genus *Malassezia spp.*

The compositions according to the invention may be intended for cosmetic or pharmaceutical and in particular dermatological application. Preferably, the compositions according to the invention are intended for cosmetic application.

The compositions according to the invention are generally applied to the scalp or the hair.

According to the mode of administration, the composition of the invention may be in any galenical form normally used, particularly in cosmetology. A preferred composition of the invention is a cosmetic composition for topical application.

The composition according to the invention, after application to human hair and scalp, may be left in or rinsed out with water or with shampoo. It may be in any form conventionally used in the field under consideration, for example in the form of an aqueous or oily solution or a dispersion of the lotion or serum type, emulsions of liquid or semi-liquid consistency of the milk type, obtained by dispersing a fatty phase in an aqueous phase (O/W) or conversely (W/O), or suspensions or emulsions of soft consistency of the cream or aqueous or anhydrous gel type, or alternatively microcapsules, microparticles or vesicular dispersions of ionic and/or nonionic type. These compositions are prepared according to the usual methods.

The composition that may be used according to the invention may also be a haircare composition, and especially a shampoo, a hairsetting lotion, a treating lotion, a styling cream or gel, a dye composition (especially oxidation dyes) optionally in the form of colouring shampoos, hair restructuring lotions, a permanent-waving composition (especially a composition for the first stage of a permanent-waving operation), a preparation (lotion, gel or shampoo) for combating hair loss, an antiparasitic shampoo.

The amounts of the various constituents in the compositions that may be used according to the invention are those conventionally used in the fields under consideration.

The compositions that may be used according to the invention may also consist of solid preparations constituting soaps or cleansing bars.

The compositions that may be used according to the invention may also be packaged in the form of an aerosol composition also comprising a propellant under pressure.

The compositions according to the invention contain a physiologically acceptable medium. In particular, this medium contains an aqueous phase containing water and optionally at least one water-miscible organic solvent, for instance C₂-C₆ monoalcohols such as ethanol, isopropanol or n-butanol, polyols, for instance propylene glycol or glycerol, or glycol ethers. This medium may contain an oily phase containing one or more fatty substances that are liquid at room temperature (25°C) and atmospheric pressure, and which are water-immiscible, known as "oils".

The physiologically acceptable medium may also comprise at least one surfactant chosen from nonionic surfactants, anionic surfactants, cationic surfactants and amphoteric surfactants, and mixtures thereof.

The compositions according to the invention may also contain as washing base at least one surfactant chosen from anionic, nonionic and amphoteric surfactants, and mixtures thereof.

As anionic surfactants that may be used in the present invention, mention may be made especially of salts, in particular alkali metal salts such as sodium salts, ammonium salts, amine salts, amino alcohol salts or alkaline-earth metal salts, for example magnesium salts, of the following types: alkyl sulfates, alkyl ether sulfates, alkylamido ether sulfates, alkylaryl-polyether sulfates, monoglyceride sulfates; alkyl sulfonates, alkylamide sulfonates, alkylaryl sulfonates, α-olefin sulfonates, paraffin sulfonates, alkyl sulfosuccinates, alkyl ether sulfosuccinates, alkylamide sulfosuccinates, alkyl sulfoacetates, acyl sarcosinates and acyl glutamates, the alkyl and acyl groups of all these compounds containing from 6 to 24 carbon atoms, and the aryl group preferably denoting a phenyl or benzyl group, and mixtures thereof.

It is also possible to use as anionic surfactant monoesters of C₆₋₂₄ alkyl and of polyglycosidedicarboxylic acids, such as alkyl glucoside citrates, polyalkyl glycoside tartrates and polyalkyl glycoside sulfosuccinates, alkyl sulfosuccinamates, acyl isethionates and N-acyltaurates, the alkyl or acyl group of all these compounds containing from 12 to 20 carbon atoms, and mixtures thereof.

Another group of anionic surfactants that may be used in the composition of the present invention is that of acyl lactylates in which the acyl group contains from 8 to 20 carbon atoms.

In addition, mention may also be made of alkyl-D-galactosiduronic acids and salts thereof, and also polyoxyalkylenated (C₆₋₂₄ alkyl)ether-carboxylic acids, polyoxyalkylenated (C₆₋₂₄ alkyl) (C₆₋₂₄ aryl) ethercarboxylic acids and polyoxyalkylenated (C₆₋₂₄ alkyl)amidoether-carboxylic acids and salts thereof, in particular those comprising from 2 to 50 ethylene oxide units, and mixtures thereof.

Alkyl sulfates, alkyl ether sulfates and alkyl ether carboxylates, and mixtures thereof, are preferably used as anionic surfactant, in particular in the form of alkali metal, alkaline-earth metal, ammonium, amine or amino alcohol salts.

As amphoteric surfactants that may be used in the present invention, mention may especially be made of aliphatic secondary or tertiary amine derivatives in which the aliphatic group is a linear or branched chain containing from 8 to 22 carbon atoms and containing at least one anionic group, for example a carboxylate, sulfonate, sulfate, phosphate or phosphonate group. Mention may also be made of (C₈-C₂₀) alkylbetaines, sulfobetaines, (C₈-C₂₀) alkylamido (C₆-C₈) alkylbetaines or (C₈-C₂₀) alkylamido (C₆-C₈) alkylsulfobetaines, and mixtures thereof.

Among the amine derivatives, mention may be made of the products sold under the name Miranol®, as described in US patents 2 528 378 and 2 781 354 and classified in the CTFA dictionary, 3rd edition, 1982, under the names Amphocarboxyglycinate and Amphocarboxypropionate, having the respective structures (2) and (3):

R₁-CONHCH₂CH₂-N (R₂) (R₃) (CH₂COO⁻) (2)

in which:
- R₁ represents an alkyl group derived from an acid R₁-COOH present in hydrolysed coconut oil, a heptyl, nonyl or undecyl group,
- R₂ represents a β-hydroxyethyl group, and
- R₃ represents a carboxymethyl group; and

   R₁'-CONHCH₂CH₂-N(B) (C) (3)

   in which:
   - B represents -CH₂CH₂OX',
   - C represents -(CH₂)_{z}-Y', with z = 1 or 2,
   - X' represents a -CH₂CH₂-COOH group or a hydrogen atom,
   - Y' represents -COOH or a -CH₂-CHOH-SO₃H group,
   - R₁' represents an alkyl group of an acid R₁'-COOH present in coconut oil or in hydrolysed linseed oil, an alkyl group, in particular a C₁₇ alkyl group and its iso form, an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, cocoamphodipropionic acid.

By way of example, mention may be made of the cocoamphodiacetate sold by the company Rhodia under the trade name Miranol^{®} C2M concentrate.

Among the amphoteric surfactants that are preferably used are (C₈₋₂₀ alkyl) betaines, (C₈₋₂₀ alkyl) amido (C₆₋₈ alkyl)betaines and alkylamphodiacetates, and mixtures thereof.

As nonionic surfactants that may be used in the composition according to the invention, mention may be made of the known compounds described especially in the book "Handbook of Surfactants" by M.R. Porter, published by Blackie & Son (Glasgow and London), 1991, pp. 116-178). They are chosen in particular from polyethoxylated, polypropoxylated or polyglycerolated fatty acids, (C₁-C₂₀)alkylphenols, α-diols or alcohols having a fatty chain containing, for example, 8 to 18 carbon atoms, it being possible for the number of ethylene oxide or propylene oxide groups to range in particular from 2 to 50 and for the number of glycerol groups to range in particular from 2 to 30, and mixtures thereof.

As nonionic surfactants that may be used in the invention, mention may also be made of condensates of ethylene oxide and of propylene oxide with fatty alcohols; polyethoxylated fatty amides preferably having from 2 to 30 ethylene oxide units; polyglycerolated fatty amides containing on average 1 to 5, and in particular 1.5 to 4, glycerol groups; ethoxylated fatty acid esters of sorbitan having from 2 to 30 ethylene oxide units; fatty acid esters of sucrose; fatty acid esters of polyethylene glycol; (C₆₋₂₄ alkyl) polyglycosides; N-(C₆₋₂₄ alkyl) glucamine derivatives; amine oxides such as (C₁₀-C₁₄) alkylamine oxides or N-(C₁₀₋₁₄ acyl)aminopropylmorpholine oxides; and mixtures thereof.

Among the nonionic surfactants mentioned above, (C₆₋₂₄ alkyl)polyglycosides are preferably used.

According to one particular embodiment of the invention, the washing base contains at least one anionic surfactant and at least one amphoteric or nonionic surfactant.

The total amount of surfactant is generally within the range from 0.01% to 50% by weight and preferably from 0.1% to 25% by weight relative to the total weight of the composition.

In particular, when the composition of the invention is in the form of a shampoo, the total amount of surfactants (or washing base) is chosen in particular from 4% to 50% by weight, for example from 8% to 25% by weight, relative to the total weight of the cosmetic composition.

The composition according to the invention may also contain at least one additional ingredient conventionally used in the fields under consideration and chosen from cosmetic active principles with a beneficial effect on the scalp and/or on the hair, for instance the zinc salts of an organic acid (acetate, glycolate, lactate, gluconate or citrate) or of a mineral acid (chloride and sulfate), vitamins (E, C, B₂, B₅ and F), UV-screening agents, free-radical scavengers, preserving agents, ceramides, plant extracts and formulation additives such as anionic, nonionic, cationic or amphoteric film-forming polymers, aqueous-phase or oily-phase polymeric thickeners, aqueous-phase non-polymeric thickeners, for instance hydroxylated or non-hydroxylated fatty acid salts or amides, nacreous agents, opacifiers, dyes that are soluble in the medium, pigments, fillers, fragrances, oils of mineral, plant and/or synthetic origin, esters of fatty acids and/or of fatty alcohols, waxes, pH stabilizers, for instance acids, bases or salts, organic solvents, silicones and electrolytes, and mixtures thereof.

The amounts of the various additional ingredients of the composition according to the invention are those generally used in the fields under consideration and are in particular within the range from 0.001% to 20% of the total weight of the composition. In addition, this composition is prepared according to the usual methods.

Needless to say, a person skilled in the art will take care to select the optional additional ingredients and/or the amount thereof such that the advantageous properties of the composition according to the invention, i.e. the activity against desquamative conditions of the hair and/or the scalp, are not, or are not substantially, adversely affected by the envisaged addition.

Advantageously, the pH of the composition of the present invention is chosen in the range from 2 to 11 and preferably from 3 to 10, for example from 5 to 8.

The other agents for combating desquamative conditions of the scalp are preferably chosen from pyridinethione salts, for instance zinc pyrithione, 1-hydroxy-2-pyrrolidone derivatives, for instance piroctone and piroctone olamine; selenium sulfides, for instance selenium disulfide; climbazole, undecylenic acid; ketoconazole and ciclopirox, or mixtures thereof. In practice, the additional active agent or the mixture of additional active agents may represent from 0.001% to 10% by weight and preferentially from 0.1% to 5% by weight relative to the total weight of the composition.

The cosmetic treatment process according to the invention is particularly suitable when the said desquamative conditions of the scalp are induced by the yeast of the genus *Malassezia spp.*

The invention is illustrated in greater detail in the examples that follow. These examples shall not in any way limit the scope of the invention.

### EXAMPLES

### Example 1: Evaluation of the antifungal activity of hydroxyethylurea on a yeast characteristic of the genus Malassezia spp (Malassezia furfur)

### MATERIALS

### The microorganism:

*Malassezia furfur* (American Type Culture Collection No. 12078) is cultured on agar on a Sabouraud slope + fats.

### Culture media:

Inoculum preparation: tryptone-salt diluent
Test + Counting: Eugon LT100 neutralizing broth Sabouraud agar + fats

### Apparatus:

Incubator/Stirrer (New Brunswick): temperature = 35°C (in the pill bottle)
stirring speed = 3000 rpm Standard microbiology laboratory equipment

### METHOD

All the manipulations described below are performed under sterile conditions.

### 1. Preparation of the sample

On the day before the test, Sabouraud broth + fats is placed in a sterile glass pill bottle (screw stopper) and incubated for 18 to 24 hours at 35°C. On the day of the test, the test composition is added and the mixture is homogenized thoroughly with a vortex blender.

Growth control: a control without product is prepared under the same conditions in order to check that the microorganism is under favourable growth conditions throughout the test.

### 2. Preparation of the inoculum:

The microbial strain is subcultured onto a suitable medium. It is incubated for three days at 35°C. On the day of the test, the slope is washed with diluent: the suspension obtained has a titre of 10⁷ micro-) organisms/ml (counting is performed).

### 3. Inoculation

The inoculum thus prepared is introduced into the pill bottle (final concentration of 10⁶ microorganisms/ml). It is homogenized with a vortex blender. The pill bottle is placed in the incubator/stirrer.

### 4. Sampling and counting

After each contact time (2, 4, 6 and 24 hours), the contents of the pill bottle are homogenized with a vortex blender. Decimal dilutions are performed in Eugon LT100 broth (up to 1/10 000). These dilutions are plated out on the surface of agar Petri dishes (Sabouraud agar) using sterile spreaders. The Petri dishes are incubated for three days in an oven at 35°C.

### 5. Reading

The colonies on the dishes containing more than 20 and less than 200 colonies are counted.

### RESULTS

The above protocol was applied to hydroxyethylurea as an aqueous 36.2% solution and to the growth control, tryptone salt.

The results obtained on this preparation are collated in the table below. They are expressed as the number of microorganisms per gram of preparation.

| **Contact time (hours)** | **Aqueous 36.2% hydroxyethylurea solution** | **Growth control** | **Reduction of the *Malassezia furfur* population** |
|---|---|---|---|
| 0 | 1.8×10⁶ | 1.8×10⁶ | - |
| 2 | 2.4×10⁵ | 2.2×10⁶ | -0.96 |
| 4 | 2.4×10⁵ | 3.2×10⁶ | -1.12 |
| 6 | 8.0×10⁴ | 3.2×10⁶ | -1.60 |
| 24 | 4.0×10⁴ | 2.6×10⁶ | -1.81 |

The activity of hydroxyethylurea on *Malassezia furfur* is demonstrated within two hours of microorganism/ product contact (1-log reduction in population relative to the control). However, the efficacy reaches its maximum after 24 hours of contact, where the yeast population is reduced by 1.8 log relative to the growth control.

### Examples 2 to 7 of antidandruff shampoos

The amounts below of the various ingredients are expressed as weight percentages.

| **INGREDIENTS** | **Ex. 2** | **Ex. 3** |
|---|---|---|
| Sodium lauryl ether sulfate (2.2 EO) as an aqueous solution | 15.40 | 15.54 |
| Oxyethylenated (4 EO) sorbitan monolaurate | 6 | 6 |
| Cocoylamidopropylbetaine as an aqueous solution | 1.66 | 1.88 |
| Hydroxyethylurea | 1 | 1 |
| Glycol distearate | 2 | 1.43 |
| Sodium N-cocoylamidoethyl-N-ethoxycarboxymethylglycinate | 0.78 | 0.56 |
| Polydimethylsiloxane (MW: 250 000) | 1.5 | |
| Hexylene glycol (2-methyl-2,4-pentanediol) | 1 | |
| Oxyethylenated (20 EO) sorbitan monolaurate | 1 | 0.4 |
| Sodium benzoate | 0.5 | 0.5 |
| Fragrance | 0.5 | 0.5 |
| Polyethylene glycol dioleate (55 EO) and propylene glycol as a water-glycol solution | 0.4 | |
| Methyl, butyl, ethyl, propyl, isobutyl p-hydroxybenzoate mixture (7/57/22/14) | 0.472 | 0.48 |
| Hydroxyethylcellulose quaternized with 2,3-epoxypropyltrimethylammonium chloride | 0.36 | |
| Carboxyvinyl polymer synthesized in an ethyl acetate/cyclohexane mixture | 0.3 | 0.3 |
| Methyl p-hydroxybenzoate | 0.028 | 0.02 |
| Hydroxypropyl guar trimethylammonium chloride | | 0.075 |
| Dimethyldiallylammonium chloride/acrylamide 50/50 copolymer as a protected aqueous solution | | |
| Polydimethylsiloxane | | 2.7 |
| Propylene glycol | | 1 |
| Microbiologically clean deionized water | qs 100 | qs 100 |

| **INGREDIENTS** | **Ex. 4** | **Ex. 5** |
|---|---|---|
| Sodium lauryl ether sulfate (2.2 EO) as an aqueous solution | 15.40 | 15.54 |
| Cocoylamidopropylbetaine as an aqueous solution | 1.66 | 1.88 |
| Hydroxyethylurea | 0.5 | 0.5 |
| Zinc pyrithione as an aqueous dispersion | 0.1 | 0.1 |
| Glycol distearate | 2 | 1.43 |
| Sodium N-cocoylamidoethyl-N-ethoxycarboxymethylglycinate | 0.78 | 0.56 |
| Polydimethylsiloxane (MW: 250 000) | 1.5 | |
| Hexylene glycol (2-methyl-2,4-pentanediol) | 1 | |
| Sodium benzoate | 0.5 | 0.5 |
| Fragrance | 0.5 | 0.5 |
| Polyethylene glycol dioleate (55 EO) and propylene glycol as a water-glycol solution | 0.4 | |
| Methyl, butyl, ethyl, propyl, isobutyl p-hydroxybenzoate mixture (7/57/22/14) | 0.472 | 0.48 |
| Hydroxyethylcellulose quaternized with 2,3-epoxypropyltrimethylammonium chloride | 0.36 | |
| Carboxyvinyl polymer synthesized in an ethyl acetate/cyclohexane mixture | 0.3 | 0.3 |
| Methyl p-hydroxybenzoate | 0.028 | 0.02 |
| Hydroxypropyl guar trimethylammonium chloride | | 0.075 |
| Dimethyldiallylammonium chloride/acrylamide 50/50 copolymer as a protected aqueous solution | | |
| Polydimethylsiloxane | | 2.7 |
| Propylene glycol | | 1 |
| Microbiologically clean deionized water | qs 100 | qs 100 |

| **INGREDIENTS** | **Ex. 6** |
|---|---|
| Vitamin B3 or PP: nicotinamide | 0.1 |
| Vitamin B6 : pyridoxine hydrochloride | 0.1 |
| 1-(Hexadecyloxy)-2-octadecanol/cetyl alcohol mixture | 2.5 |
| Coconut acid monoisopropanolamide | 0.66 |
| Cocoylamidopropylbetaine as an aqueous solution | 2.4 |
| Sodium lauryl ether sulfate (2.2 EO) as an aqueous solution | 15.54 |
| Propylene glycol | 0.1 |
| Polydimethylsiloxane (MW: 250 000) | 1.995 |
| Carboxyvinyl polymer synthesized in an ethyl acetate/cyclohexane mixture | 0.29 |
| Fragrance | 0.5 |
| Stabilized (0.6% phenoxyethanol) mixture of acids extracted from fruit, sugar cane, lemon, apple and green tea in water | 0.1 |
| Pasteurized ultrafiltered concentrated apple juice with a sugar content of 65.5% | 0.5 |
| Methyl, ethyl, propyl, butyl, isobutyl p-hydroxybenzoate/2-phenoxyethanol mixture | 0.05 |
| 1,3-Dimethylol-5,5-dimethylhydantoin as an aqueous solution | 0.25 |
| Methyl p-hydroxybenzoate, sodium salt | 0.2 |
| Pure sodium hydroxide | qs |
| Hydroxyethylurea | 1 |
| Sodium chloride | 0.6 |
| Citric acid monohydrate | qs |
| Microbiologically clean deionized water | qs 100 |

| **INGREDIENTS** | **Ex. 7** |
|---|---|
| Pure sodium hydroxide | qs |
| Hydroxyethylurea | 1 |
| 3-L-Menthoxy-1,2-propanediol | 0.1 |
| Citric acid monohydrate | qs |
| Methyl, butyl, ethyl, propyl, isobutyl p-hydroxybenzoate mixture (7/57/22/14) | 0.5 |
| Sodium benzoate | 0.6 |
| Fragrance | 0.5 |
| Hydroxyethylcellulose quaternized with 2,3-epoxypropyltrimethylammonium chloride | 0.27 |
| Polyethylene glycol dioleate (55 EO) and propylene glycol as a water-glycol solution | 0.4 |
| Acrylic polymer in emulsion | 0.9 |
| Glycerol | 2 |
| Ricinoleic monoethanolamide monosulfosuccinate, disodium salt | 0.8 |
| Cocoylamidopropylbetaine/glyceryl monolaurate mixture as an aqueous 30% solution | 3.81 |
| Sodium lauryl ether sulfate (2.2 EO) as an aqueous solution | 9.1 |
| Microbiologically clean deionized water | qs 100 |

## Claims

1. Non-therapeutic use of a cosmetic composition comprising, in a physiologically acceptable medium, at least one hydroxyalkylurea corresponding to the general formula (1) : 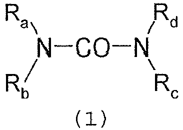 in which Rₐ, R_{b}, R_{c} and R_{d} each independently represent a hydrogen atom, a C₁-C₄ alkyl group or a C₂-C₆ hydroxyalkyl group possibly containing from 1 to 5 hydroxyl groups, at least one of the radicals Rₐ-R_{d} representing a hydroxyalkyl group, and also the salts, solvates and isomers thereof, as an agent for treating dandruff.

2. Use according to Claim 1, **characterized in that** Rₐ is a hydroxyalkyl group and R_{b}, R_{c} and R_{d} represent, independently of each other, a hydrogen atom or a C₁-C₄ alkyl group.

3. Use according to Claim 2, **characterized in that** Rₐ is a hydroxyalkyl group and R_{b}, R_{c} and R_{d} each represent a hydrogen atom.

4. Use according to any one of Claims 1 to 3, **characterized in that** the compound of formula (1) is chosen from N-(2-hydroxyethyl)urea; N-(2-hydroxypropyl)urea; N-(3-hydroxypropyl)urea; N-(2,3-dihydroxypropyl)urea; N-(2,3,4,5,6-pentahydroxyhexyl)urea; N-methyl-N-(1,3,4,5,6-pentahydroxy-2-hexyl)urea; N-methyl-N'-(1-hydroxy-2-methyl-2-propyl)urea; N-(1-hydroxy-2-methyl-2-propyl)urea; N-(1,3-dihydroxy-2-propyl)urea; N-[tris(hydroxymethyl)methyl]urea; N-ethyl-N'-(2-hydroxyethyl)urea; N,N-bis(2-hydroxyethyl)-urea; N,N'-bis(2-hydroxyethyl)urea; N,N-bis(2-hydroxypropyl)urea; N,N'-bis(2-hydroxypropyl)urea; N,N-bis(2-hydroxyethyl)-N'-propylurea; N,N-bis(2-hydroxypropyl)-N'-(2-hydroxyethyl)urea; N-tert-butyl-N'-(2-hydroxyethyl)-N'-(2-hydroxypropyl)urea; N-(1,3-dihydroxy-2-propyl)-N'-(2-hydroxyethyl)urea; N,N-bis(2-hydroxyethyl)-N',N'-dimethylurea; N,N,N',N'-tetrakis(2-hydroxyethyl)urea; N',N'-bis(2-hydroxyethyl)-N',N'-bis(2-hydroxypropyl)urea.

5. Use according to any one of Claims 1 to 4, **characterized in that** the hydroxyalkylurea is N-(2-hydroxyethyl)urea.

6. Use according to any one of Claims 1 to 4, **characterized in that** the hydroxyalkylurea is present in the composition in contents of from 0.01% to 50% by weight, more preferentially from 0.1% to 20% and even more preferentially from 0.1% to 10% by weight relative to the total weight of the composition.

7. Composition comprising, in a physiologically acceptable medium:
(a) at least one hydroxyalkylurea of formula (1) or a salt and/or solvate and/or isomer thereof as defined according to any one of the preceding claims,
(b) at least one other agent for combating desquamative conditions of the scalp.

8. Composition according to Claim 7, in which the additional active agent for combating desquamative conditions of the scalp is chosen from pyridinethione salts; 1-hydroxy-2-pyrrolidone derivatives; selenium sulfides; ketoconazole; ciclopirox, or mixtures thereof.

9. Composition according to Claim 7, in which the additional active agent for combating desquamative conditions of the scalp represents from 0.001% to 10% by weight and preferentially from 0.1% to 5% by weight relative to the total weight of the composition.

10. Composition according to any one of Claims 7 to 9, **characterized in that** the hydroxyalkylurea is present in the composition in contents of from 0.01% to 50% by weight, more preferentially from 0.1% to 20% and even more preferentially from 0.1% to 10% by weight relative to the total weight of the composition.

11. Cosmetic, non-therapeutic treatment process for combating dandruff, **characterized in that** a cosmetic composition comprising, in a physiologically acceptable medium, at least one hydroxyalkylurea of formula (1) or a salt and/or solvate and/or isomer thereof as defined according to any one of the preceding claims is applied to the hair and/or the scalp.

12. Hydroxyalkylurea of formula (1) or a salt and/or solvate and/or isomer thereof, for use as medicament for treating desquamative conditions of the scalp.

13. Hydroxyalkylurea of formula (1) or a salt and/or solvate and/or isomer thereof, for use as medicament according to claim 12, **characterized in that** the desquamative conditions of the scalp are induced by the yeast of the genus *Malassezia spp.*

## Patentansprüche

1. Nichttherapeutische Verwendung einer kosmetischen Zusammensetzung, die in einem physiologisch unbedenklichen Medium mindestens einen Hydroxyalkylharnstoff der allgemeinen Formel (1): 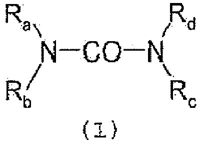 in der Rₐ, R_{b}, R_{c} und R_{d} jeweils unabhängig voneinander für ein Wasserstoffatom, eine C₁-C₄-Alkylgruppe oder eine C₂-C₆-Hydroxyalkylgruppe, die 1 bis 5 Hydroxylgruppen enthalten kann, stehen, wobei mindestens einer der Reste Rₐ-R_{d} für eine Hydroxyalkylgruppe steht, sowie die Salze, Solvate und Isomere davon umfasst, als Mittel zur Behandlung von Schuppen.

2. Verwendung nach Anspruch 1, **dadurch gekennzeichnet, dass** Rₐ für eine Hydroxyalkylgruppe steht und R_{b}, R_{c} und R_{d} jeweils unabhängig voneinander für ein Wasserstoffatom oder eine C₁-C₄-Alkylgruppe stehen.

3. Verwendung nach Anspruch 2, **dadurch gekennzeichnet, dass** Rₐ für eine Hydroxyalkylgruppe steht und R_{b}, R_{c} und R_{d} jeweils für ein Wasserstoffatom stehen.

4. Verwendung nach einem der Ansprüche 1 bis 3, **dadurch gekennzeichnet, dass** die Verbindung der Formel (1) aus N-(2-Hydroxyethyl)harnstoff; N-(2-Hydroxypropyl)harnstoff; N-(3-Hydroxypropyl)harnstoff; N-(2,3-Dihydroxypropyl)harnstoff; N-(2,3,4,5,6-Pentahydroxyhexyl)harnstoff; N-Methyl-N-(1,3,4,5,6-pentahydroxy-2-hexyl)harnstoff; N-Methyl-N'-(1-hydroxy-2-methyl-2-propyl)harnstoff; N-(1-Hydroxy-2-methyl-2-propyl)harnstoff; N-(1,3-Dihydroxy-2-propyl)harnstoff; N-[Tris(hydroxymethyl)methyl]harnstoff; N-Ethyl-N'-(2-hydroxyethyl)harnstoff; N,N-Bis(2-hydroxyethyl)harnstoff; N,N'-Bis(2-hydroxyethyl)harnstoff; N,N-Bis(2-hydroxypropyl)harnstoff; N,N'-Bis(2-hydroxypropyl)harnstoff; N,N-Bis(2-hydroxyethyl)-N'-propylharnstoff; N,N-Bis(2-hydroxypropyl)-N'-(2-hydroxyethyl)harnstoff; N-tert-Butyl-N'-(2-hydroxyethyl)-N'-(2-hydroxypropyl)harnstoff; N-(1,3-Dihydroxy-2-propyl)-N'-(2-hydroxyethyl)harnstoff; N,N-Bis(2-hydroxyethyl)-N',N'-dimethylharnstoff; N,N,N',N'-Tetrakis(2-hydroxyethyl)harnstoff und N',N'-Bis(2-hydroxyethyl)-N',N'-bis(2-hydroxypropyl)harnstoff ausgewählt ist.

5. Verwendung nach einem der Ansprüche 1 bis 4, **dadurch gekennzeichnet, dass** es sich bei dem Hydroxyalkylharnstoff um N-(2-Hydroxyethyl)harnstoff handelt.

6. Verwendung nach einem der Ansprüche 1 bis 4, **dadurch gekennzeichnet, dass** der Hydroxyalkylharnstoff in der Zusammensetzung in Gehalten von 0,01 bis 50 Gew.-%, weiter bevorzugt von 0,1 bis 20 Gew.-% und noch weiter bevorzugt 0,1 bis 10 Gew.-%, bezogen auf das Gesamtgewicht der Zusammensetzung, vorliegt.

7. Zusammensetzung, die in einem physiologisch unbedenklichen Medium:
(a) mindestens einen Hydroxyalkylharnstoff der Formel (1) oder ein Salz und/oder Solvat und/oder Isomer davon gemäß einem der vorhergehenden Ansprüche,
(b) mindestens ein anderes Mittel zur Bekämpfung von desquamativen Zuständen der Kopfhaut umfasst.

8. Zusammensetzung nach Anspruch 7, wobei das zusätzliche aktive Mittel zur Bekämpfung von desquamativen Zuständen der Kopfhaut aus Pyridinethionsalzen; 1-Hydroxy-2-pyrrolidonderivaten; Selensulfiden; Ketoconazol; Ciclopirox oder Mischungen davon ausgewählt ist.

9. Zusammensetzung nach Anspruch 7, wobei das zusätzliche aktive Mittel zur Bekämpfung von desquamativen Zuständen der Kopfhaut 0,001 bis 10 Gew.-% und vorzugsweise 0,1 bis 5 Gew.-%, bezogen auf das Gesamtgewicht der Zusammensetzung, ausmacht.

10. Zusammensetzung nach einem der Ansprüche 7 bis 9, **dadurch gekennzeichnet, dass** der Hydroxyalkylharnstoff in der Zusammensetzung in Gehalten von 0,01 bis 50 Gew.-%, weiter bevorzugt von 0,1 bis 20 Gew.-% und noch weiter bevorzugt 0,1 bis 10 Gew.-%, bezogen auf das Gesamtgewicht der Zusammensetzung, vorliegt.

11. Verfahren zur kosmetischen, nichttherapeutischen Behandlung zur Bekämpfung von Schuppen, **dadurch gekennzeichnet, dass** man eine kosmetische Zusammensetzung, die in einem physiologisch unbedenklichen Medium mindestens einen Hydroxyalkylharnstoff der Formel (1) oder ein Salz und/oder Solvat und/oder Isomer davon gemäß einem der vorhergehenden Ansprüche umfasst, auf das Haar und/oder die Kopfhaut aufbringt.

12. Hydroxyalkylharnstoff der Formel (1) oder ein Salz und/oder Solvat und/oder Isomer davon zur Verwendung als Medikament zur Behandlung von desquamativen Zuständen der Kopfhaut.

13. Hydroxyalkylharnstoff der Formel (1) oder ein Salz und/oder Solvat und/oder Isomer davon zur Verwendung als Medikament nach Anspruch 12, **dadurch gekennzeichnet, dass** die desquamativen Zustände der Kopfhaut durch die Hefe der Gattung Malassezia spp. induziert werden.

## Revendications

1. Utilisation non thérapeutique d'une composition cosmétique comprenant dans un milieu physiologiquement acceptable au moins une hydroxyalkylurée répondant à la formule générale (1) : 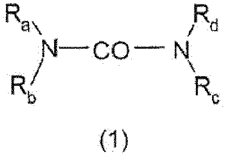 dans laquelle Rₐ, R_{b}, R_{c}, et R_{d} représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux Rₐ-R_{d} représentant un groupe hydroxyalkyle, ainsi que ses sels, solvats et isomères, comme agent de traitement des pellicules.

2. Utilisation selon la revendication 1, **caractérisée en ce que** Rₐ est un groupe hydroxyalkyle et R_{b}, R_{c}, et R_{d} représentent, indépendamment les uns des autres, un atome d'hydrogène ou un groupe alkyle en C₁-C₄.

3. Utilisation selon la revendication 2, **caractérisée en ce que** Rₐ est un groupe hydroxyalkyle et R_{b}, R_{c}, et R_{d} représentent chacun un atome d'hydrogène.

4. Utilisation selon l'une quelconque des revendications 1 à 3, **caractérisée en ce que** le composé de formule (1) est choisi parmi la N-(2-hydroxyéthyl)-urée ; la N-(2-hydroxypropyl)-urée ; la N-(3-hydroxypropyl)-urée ; la N-(2,3-dihydroxypropyl)-urée ; la N-(2,3,4,5,6-pentahydroxyhexyl)-urée ; la N-méthyl-N-(1,3,4,5,6-pentahydroxy-2-hexyl)-urée ; la N-méthyl-N'-(1-hydroxy-2-méthyl-2-propyl)-urée ; la N-(1-hydroxy-2-méthyl-2-propyl)-urée ; la N-(1,3-dihydroxy-2-propyl)-urée ; la N-(tris-hydroxyméthyl-méthyl)-urée ; la N-éthyl-N'-(2-hydroxyéthyl)-urée ; la N,N-bis-(2-hydroxyéthyl)-urée ; la N,N'-bis-(2-hydroxyéthyl)-urée ; la N,N-bis-(2-hydroxypropyl)-urée ; la N,N'-bis-(2-hydroxypropyl)-urée ; la N,N-bis-(2-hydroxyéthyl)-N'-propyl-urée ; la N,N-bis-(2-hydroxypropyl)-N'-(2-hydroxyéthyl)-urée ; la N-tert-butyl-N'-(2-hydroxyéthyl)-N'-(2-hydroxypropyl)-urée ; la N-(1,3-dihydroxy-2-propyl)-N'-(2-hydroxyéthyl)-urée ; la N,N-bis-(2-hydroxyéthyl)-N',N'-dimethyl-urée ; la N,N,N',N'-tetrakis-(2-hydroxyéthyl)-urée ; la N',N'-bis-(2-hydroxyéthyl)-N',N'-bis-(2-hydroxypropyl)-urée.

5. Utilisation selon l'une quelconque des revendications 1 à 4, **caractérisée en ce que** l'hydroxyalkylurée est la N-(2-hydroxyéthyl)-urée.

6. Utilisation selon l'une quelconque des revendications 1 à 4, **caractérisée en ce que** l'hydroxyalkylurée est présente dans la composition à des teneurs de 0,01 à 50% en poids, plus préférentiellement de 0,1 à 20 % et encore plus préférentiellement de 0,1 à 10% en poids par rapport au poids total de la composition.

7. Composition comprenant dans un milieu physiologiquement acceptable :
(a) au moins une hydroxyalkylurée de formule (1) ou l'un de ses sels et/ou de ses solvats et/ou de ses isomères telle que définie selon l'une quelconque des revendications précédentes,
(b) au moins un autre agent contre les états desquamatifs du cuir chevelu.

8. Composition selon la revendication 7, où l'agent additionnel actif contre les états desquamatifs du cuir chevelu est choisi parmi les sels de pyridinethione ; les dérivés de 1-hydroxy-2-pyrrolidone ; les sulfures de sélénium ; le kétoconazole ; le ciclopirox ou leurs mélanges.

9. Composition selon la revendication 7, où l'agent additionnel actif contre les états desquamatifs du cuir chevelu représente de 0,001% à 10% en poids et préférentiellement de 0,1 à 5% en poids par rapport au poids total de la composition.

10. Composition selon l'une quelconque des revendications 7 à 9, **caractérisée en ce que** l'hydroxyalkylurée est présente dans la composition à des teneurs de 0,01 à 50% en poids, plus préférentiellement de 0,1 à 20% et encore plus préférentiellement de 0,1 à 10% en poids par rapport au poids total de la composition.

11. Procédé de traitement cosmétique non thérapeutique pour lutter contre les pellicules, **caractérisé en ce qu'**on applique sur les cheveux et/ou le cuir chevelu une composition cosmétique comprenant dans un milieu physiologiquement acceptable au moins une hydroxyalkylurée de formule (1) ou l'un de ses sels et/ou de ses solvats et/ou de ses isomères telle que définie selon l'une quelconque des revendications précédentes.

12. Hydroxyalkylurée de formule (1) ou l'un de ses sels et/ou de ses solvats et/ou de ses isomères pour utilisation comme médicament pour traiter les états desquamatifs du cuir chevelu.

13. Hydroxyalkylurée de formule (1) ou l'un de ses sels et/ou de ses solvats et/ou de ses isomères pour utilisation comme médicament selon la revendication 12, **caractérisée en ce que** les états desquamatifs du cuir chevelu sont induits par la levure du genre *Malassezia spp.*
